# EUROPEAN PATENT SPECIFICATION

(11) **EP 2 564 197 B1**
(45) Date of publication and mention of the grant of the patent: **18.03.2015**
(21) Application number: 11715922.8
(22) Date of filing: 21.04.2011
(51) Int. Cl.: G01N 33/569, G01N 33/68

(54) **A METHOD FOR DETECTING THE SUSCEPTIBILITY TO DEVELOP ADVERSE SIDE EFFECTS RELATED TO BIOIMPLANTS**
VERFAHREN FÜR DEN NACHWEIS DER PRÄDISPOSITION ZUR ENTWICKLUNG VON NEBENWIRKUNGEN IM ZUSAMMENHANG MIT BIOIMPLANTATEN
MÉTHODE DE DÉTECTION DE LA SUSCEPTIBILITÉ DE DÉVELOPPER DES EFFETS SECONDAIRES NÉFASTES SUITE À DES IMPLANTS BIOLOGIQUES.

(30) Priority: 27.04.2010 ES 201030604
(43) Date of publication of application: 06.03.2013
(73) Proprietor: Fundació Hospital Universitari Vall d'Hebron - Institut de Recerca, 08035 Barcelona (ES)
(72) Inventor: SCHWARTZ NAVARRO, Simón, E-08035 Barcelona (ES); ALIJOTAS REIG, Jaume, E-08035 Barcelona (ES)
(74) Representative: ZBM Patents - Zea, Barlocci & Markvardsen
(86) International application number: PCT/EP2011/056393
(87) International publication number: WO 2011/134879

(56) References cited:
- GIMBEL H V ET AL: "Adverse response to phakic IOLs possibly related to human leukocyte antigen", CLINICAL & SURGICAL OPHTHALMOLOGY, MEDIACONCEPT, MONTREAL, CA, vol. 24, no. 12, 1 January 2006 (2006-01-01), pages 490-492, XP008138241, ISSN: 1705-4842
- TRENTHAM D E: "Adverse reactions to bovine collagen implants. Additional evidence for immune response gene control of collagen reactivity in humans", ARCHIVES OF DERMATOLOGY, AMERICAN MEDICAL ASSOCIATION, US, vol. 122, no. 6, 1 June 1986 (1986-06-01), pages 643-644, XP008138242, ISSN: 0003-987X, DOI: DOI:10.1001/ARCHDERM.122.6.643
- MEIER LOTHAR G ET AL: "Development of polyarthritis after insertion of silicone breast implants followed by remission after implant removal in 2 HLA-identical sisters bearing rheumatoid arthritis susceptibility genes", JOURNAL OF RHEUMATOLOGY, JOURNAL OF RHEUMATOLOGY PUBLISHING COMPANY, CA, vol. 24, no. 9, 1 January 1997 (1997-01-01), pages 1838-1841, XP008138244, ISSN: 0315-162X
- SELVA-O'CALLAGHAN A ET AL: "Silicone gel filled breast implants and dermatomyositis", CLINICAL AND EXPERIMENTAL RHEUMATOLOGY, PACINI, PISA, IT, vol. 22, no. 3, 1 May 2004 (2004-05-01), page 376, XP008138243, ISSN: 0392-856X
- DI LORENZO GABRIELE ET AL: "Morphea after silicone gel breast implantation for cosmetic reasons in an HLA-B8,DR3-positive woman", INTERNATIONAL ARCHIVES OF ALLERGY AND IMMUNOLOGY, KARGER AG, CH, vol. 112, no. 1, 1 January 1997 (1997-01-01), pages 93-95, XP008138237, ISSN: 1018-2438, DOI: DOI:10.1159/000237437 cited in the application
- LAPPE ET AL: "Silicone-reactive disorder: A new autoimmune disease caused by immunostimulation and superantigens", MEDICAL HYPOTHESES, EDEN PRESS, PENRITH, US, vol. 41, no. 4, 1 October 1993 (1993-10-01), pages 348-352, XP023026349, ISSN: 0306-9877, DOI: DOI:10.1016/0306-9877(93)90081-Z [retrieved on 1993-10-01]
- WILSON S E ET AL: "Graft failure after penetrating keratoplasty", SURVEY OF OPHTHALMOLOGY, SURVEY OF OPHTHALMOLOGY INC, XX, vol. 34, no. 5, 1 March 1990 (1990-03-01), pages 325-356, XP026344899, ISSN: 0039-6257 [retrieved on 1990-03-01]
- BRAUN ET AL: "112-P: Report of a new HLA-DRB1*03 allele escaping detection by high-resolution sequence-specific priming and sequence-based typing", HUMAN IMMUNOLOGY, NEW YORK, NY, US, vol. 68, no. 1, 22 September 2007 (2007-09-22), page S71, XP022265093, ISSN: 0198-8859, DOI: 10.1016/J.HUMIMM.2007.08.135

## Description

The present invention relates to the field of medicine, namely to the field of adverse effects derived from bioimplants which are applied or inserted into the body mainly for aesthetic reasons (cosmetic or therapeutic). The invention is framed in the field of health maintenance and provides for tools to analyse the risk of severe side effects derived from the application of said bioimplants.

### BACKGROUND ART

An ever-increasing number of people seek medical solutions for their ageing skin or for purely aesthetic and cosmetic indications. Others, due to pathological conditions turn also to the use of bioimplants. A bioimplant is defined as a biomaterial surgically implanted in a person's body to replace damaged tissue. The term bioimplant defines a kind of implant, which generally is associated to a non-metallic implant. Common areas of application of said bioimplants include orthopedic (especially maxillofacial) re-constructive prosthesis, breast augmentation prosthesis, cardiac prostheses (artificial heart valves like the Chitra heart valve), skin and cornea. One kind of bioimplant broadly used nowadays are the polymeric or ceramic implants, being an example of them the implant fillers or dermal and sub-dermal microprotheses.

Currently, physicians may use many different types of dermal and sub-dermal fillers, including non-permanent, permanent, reversible or non-reversible materials. The dermal and sub-dermal fillers are materials to be injected in order to correct wrinkles, expression lines, lipodistrophy and other traits that an individual may want or may need to correct, mainly in the face. These compounds are used to fill the cavities or grooves created in the dermal tissue due to different causes. Different classifications of fillers have been proposed, according to their origin and longevity (duration). At present, the most filler used worldwide are: acrylamides (polyacrylamide and polyalkylimide compounds), hyaluronic acid compounds, polylactic acid, polymethylmethacrylate compounds, calcium hydroxyapatite, collagen, polytetrafluoroethylene and silicone oil. Although manufacturers and different publications claim that the fillers are non-toxic and non-immunogenic and that complications are very uncommon, unwanted side effects occur with all compounds used. Some of these side effects are late-onset side effects, which in many cases are difficult to be related with the use of said fillers or bioimplants. The adverse side effects associated to the injection of dermal or sub-dermal fillers may be severe side effects, leading to chronic granulomatous conditions and rarely to autoimmune diseases. Thus, two notions are lacking in the usual classification of fillers used: long term safety and reversibility of side effects.

When a dermal filler material is injected normal foreign body-type reactions (the so called "stable granulomas") occur. Unfortunately, in some people these stable granulomas develop into persistent inflammatory harmful granulomas, granulomatous disorders or, although rarely, into autoimmune diseases. Sometimes, undesirable side effects appear during minor trauma or after infection. It is hypothesised that the genetic background (own and specific of every individual) must play an important role in the development of these adverse reactions but its exact cause is still unknown and no predictions can be made. This fact represents a real drawback due to the ever-increasing number of people turning to the use of these kind of bioimplants.

Among the patients that present intermediate or delayed adverse effects related to dermal fillers, most of them report local or regional side effects, mainly related to connective tissue diseases. Noteworthy is that several of the patients present systemic, inflammatory, immune-mediated disorders, such us scleroderma or scleroderma-like disease, polymyositis, Sjögren syndrome, cutaneous sarcoidosis, granulomatous disorders, pneumonitis and human adjuvant disease.

One approach to previously detect if an individual is susceptible of presenting intermediate or delayed (late-onset) adverse effects related to dermal and sub-dermal fillers, consists in injecting 0.1 ml of filler intradermally injected into the forearm skin and visualize if any reaction exists. However, the time lapse until appearance of some reactions is usually of 5-8 weeks. Additionally, for the biopsy provum (test) the skin is injured. Moreover, Health authorities disagree with this procedure. Thus, there is a need of other ways to detect if a patient will adversely react to the implanted filler.

The human leukocyte antigen system (HLA) is the name of the human major histocompatibility complex (MHC). It is a superlocus that contains a large number of genes related to immune system function in humans. All these genes are clustered on a 4 Mb-segment of the short arm of chromosome 6 in humans. The HLA region comprises six major loci that encode structurally homologous proteins which are classified into HLA class I (HLA-A, B, Cw) and class II (HLA-DR, DQ, DP), that present antigens to two different subsets of cells.

Class I molecules consist of two polypeptide chains, α and β2-microglobulin. The two chains are linked noncovalently via interaction of β2-microglobulin and the α3 domain. Only the α chain is polymorphic and encoded by a HLA gene, while the β2-microglobulin subunit is not polymorphic and encoded by the Beta-2 microglobulin gene. The α3 domain is plasma membrane spanning and interacts with the transmembrane glycoprotein CD8 co-receptor of T-cells. The α1 and α2 domains fold to make up a groove for peptides to bind. MHC class I molecules bind peptides that are 8-10 amino acid in length.

In the case of the HLA-B locus, the polymorphisms are mainly located in exons 2 and 3. To date, hundreds of allelic variants of this locus are known, which means that this locus is the most polymorphic of HLA loci. The allelic variations of the HLA-B locus are the most important in the selection of the putative transplant within class I molecules. Each of the allelic variant is given a particular number (such as HLA-B*08). Closely related alleles are categorized together; for example, at least 54 very similar alleles are subtypes of HLA-B*08. These subtypes are designated as HLA-B*080101 to HLA-B*0854. The HLA-B gene has many different normal variations, allowing each person's immune system to react to a wide range of foreign invaders.

Class II molecules are heterodimeric glycoproteins consisiting of a non-polymorphic alpha chain and a polymorphic beta chain. Class II HLA (or MHC) molecules are expressed on the cell surface of macrophages, B-cells, activated T-cells, and other cell types involved in presenting antigens to T cells that express the CD4 cell surface glycoprotein. DP, DQ and DR genes are located in separated regions of the MHC. In the DR region, a single DRA locus encodes the non-polymorphic DRalpha chain, but nine different DRB loci, termed DRB1 to DRB9, encode the polymorphic DRbeta chain. Moreover, the number of identified DRB alleles is continuously increasing.

In the case of HLA-DRB1 locus, different alleles of this locus are called DRB1*01, DRB1*02, DRB1*03 etc., and these alleles all together form what is called an "allelic group" in this description. Moreover, several genetic sequences are grouped under a specific DRB1 allele. Thus, the allele DRB1*03 can have different sequences which are named DRB1*0301, DRB1*0302, DRB1*031101, DRB1*031102, etc.

The genes that encode the human leukocyte antigens (HLA) system are among the most variable genes in humans, and each variant codes for molecules which bind to different set of peptides. These genes are the same in all the cells of a particular individual, but differ from person to person.

Although the inter-individuals allelic variability of the HLA has been deeply investigated and some genetic markers have been discovered allowing the performance of tests to avoid organ transplant rejection, only minor non-representative data exist correlating the genetic predisposition of an individual to develop adverse effects related to materials implanted into the body.

Several studies have tried to link many systemic autoimmune or granulomatous diseases with different haplotypes of HLA (MHC), especially class I and class II antigens. Thus, in the document Di Lorenzo et al., "Morphea after Silicone Gel Breast Implantation for Cosmetic Reasons in an HLA-BS, DR3-Positive Woman, Int. Arch Allergy Immunol 1997, vol. 112, pp. 93-95, the authors disclose a single HLA-B*08, DR3-positive patient with localized morphea after silicone gel breast implantation. The authors suggest that there is a genetic background and that the above-mentioned haplotype is involved in the autoimmune response, which could be propitiated by the silicone bioimplant. Moreover, the authors consider that the data do not support a clear relationship between the haplotype and the observed effects, and that further studies are required. In fact, the present case is one of the small number of cases in which a silicon implant seems to produce adverse effects, namely an autoimmune reaction, and no statistical significance can be attributed to the same.

Several documents refer to the use of antigens of the major histocompatibility (MHC) complex of an individual to predict whether the individual will develop adverse effects to a bioimplant or not. Document of Gimbel et al., "Adverse response to phakic IOLs possibly related to human leukocyte antigen", Clinical & Surgical Ophtalmology - 2006, Vol. No. 24(12), pp.: 490-492, discloses that the HLA-B27 region can be used to predict susceptibility to bioimplants, namely to implantable corrective lens, in particular to predict the susceptibility to uveitis. On the other side, the document Tretham et al., "Adverse reactions to bovine collagen implants. Additional evidence for immune response gene control of collagen reactivity in humans", Archives of dermatology - 1986, Vol. No. 122(6), pp.: 643-644, discloses the use of different alleles of HLA to predict compatibility to collagen implants.

Intolerance for silicon breast implants appears related to different HLA-antigens as disclosed in document Meier et al., "Development of polyarthritis after insertion of silicone breast implants followed by remission after implant removal after implant removal in 2 HLA-identical sisters bearing rheumatoid arthritis susceptibility genes", The Journal of Rheumatology -1997, Vol. No. 24(9), pp.: 1838-1841. Meier et al, show that HLA-DRB1*0405 and HLA-DQB1*0302 (associated with RA) and HLA-DRB4*01 (associated with mixed connective tissue disease and autoimmune reactions) could be the cause of neurologic symptoms and of polyarticular arthritis in two patients with silicone breast implants, since after removal of the implants the symptoms disappeared or were diminished. Also the document of Selva-O'Callaghan et al., "Silicone gel filled breast implants and dermatomyositis", Clinical Experimental Rheumatology - 2004, Vol. No. 22(3), pp.: 376 refers to two patients from which the authors speculate that the identical class II HLA alleles resulted in a reaction to the silicone that triggered humoral autoimmune responses. Further, the document Lappe et al., "Silicone-reactive disorder: A new autoimmune disease caused by immunostimulation and superantigens", Medical Hypotheses- 1993, Vol. No. 43, pp.: 348-352, discloses that the HLA-B8 antigen can be relevant to develop intolerance to silicon breast implants, although the authors indicate that this stills need to be shown. Thus, a meaningful correlation and the assessment with other implants is needed.

Further, the document of Wilson et al., "Graft failure after penetrating keratoplasty", Surv. Opthalmol. -1990, Vol. No. 34(5), pp.: 325-356 tells possible causes of graft failures after keratoplasty, including donor tissue status and surgery methods, but no mention is done to allelic factors.

The document Braun et al., "Report of a new HLA-DRB1*03 allele escaping detection by high-resolution sequence-specific priming and sequence-based typing", Human Immunology-2007, Vol. No. 68(1), page S71, discloses a new identified allele differing from HLA-DRB1*030101 by several nucleotide replacements. No correlation with bioimplants is done.

Silicone used as filler is a gel consisting in polymerized siloxanes. Although widely used since 1960, the secondary effects derivable from its use as filler are lower in type and number of cases than those observed when other fillers have been used as bioimplants, such as those associated with the use of the organic bioimplants, i.e. collagen, polylactic-L-acid, polyacrylamide, polyalkylimide, hyaluronic acid, polytetrafluoroethylene, methacrylate implants as well as the inorganic bioimplant made of microspheres of calcium-hydroxyapatite. All these materials have different structural features, thus, it is desirable to find a common pattern allowing to correlate the genetic predisposition to rejection of an implant or to develop adverse side effects for all bioimplants.

### SUMMARY OF THE INVENTION

The inventors, suspecting that probably, in a predisposed host, the likelihood to develop different hypersensitivity or type IV immune-mediated disorders may be related to diverse and repetitive antigenic exposure, broadly tested the genotypes of patients which presented severe adverse side effects (adverse effects) after the implantation of a material, namely a bioimplant, such as a dermal or sub-dermal filler.

The invention provides, in a first aspect, an *in vitro* method for the analysis of the genetic predisposition of an individual to develop adverse effects related to non-metallic bioimplants implanted into the body, comprising determining, in a biological sample from said individual,
the presence of the antigen HLA-B*08 of the major histocompatibility complex; and wherein the presence of at least said antigen is indicative of genetic predisposition to develop said adverse effects.

The method of the invention represents a goal to outstanding drawbacks of the state of the art since it allows the individual and the practitioner considering all the factors before the implantation of the non-metallic materials, namely bioimplants, such as dermal or sub-dermal fillers, into the body. Thus, the method of the invention is a useful assay for previously detecting, in an innocuous matter, the probability of an individual to develop adverse side effects when to said individual a bioimplant is applied.

The invention provides also the use of a kit for carrying out the method as defined above, wherein the kit comprises adequate means for determining the presence of the antigen HLA-B*08 or the presence of the antigens HLA-B*08 and HLA-DRB1*03 of the major histocompatibility complex.

Further, it is also an aspect of the invention the use of the antigen HLA-B*08, as marker for the analysis of the genetic predisposition of an individual to develop adverse effects related to non-metallic bioimplants implanted into the body.

Another aspect of the invention is the use of the antigens HLA-B*08 and HLA-DRB1*03, as markers for the analysis of the genetic predisposition of an individual to develop adverse effects related to non-metallic bioimplants implanted into the body.

### DETAILED DESCRIPTION OF EMBODIMENTS OF THE INVENTION

The following definitions are provided for the purpose of understanding:
The terms "adverse effect" and "adverse side effect" are used as synonymous in this description and they refer to any harmful and/or undesired effect resulting from a medication or other intervention such as surgery. An adverse effect may be termed a "side effect", when judged to be secondary to a main or therapeutic effect.

The terms "genotyping" and "typing" are used as synonymous in this description and they refer to any testing that reveals the specific alleles inherited by an individual, particularly useful for situations in which more than one genotypic combination can produce the same clinical presentation. In the present description, the term "locus" means the position occupied by each HLA gene (e.g. B locus of HLA). "Loci" (plural of locus) for DR antigens include DRA and DRB1-9.

The term "allele" refers to one of two or more alternative forms of a gene, differing in genetic sequence and resulting in observable differences in heritable character (phenotype), and they are found at the same place on a chromosome.

"Linkage" describes the tendency of genes, alleles, loci or genetic markers to be inherited together because of their location on the same chromosome. They can be measured by percent recombination between the two genes, alleles, loci or genetic markers that are physically-linked on the same chromosome.

In the sense of this description, "alteration" of the gene means any structural change in the nucleotide sequence considered as wild-type. Examples of alterations can include a single nucleotide polymorphism, a deletion, an insertion, a substitution or a duplication of one or more nucleotides, and a chemical modification on a nucleotide (e.g. methylation).

"Linkage disequilibrium (LD) or "allelic association" means the preferential association of a particular allele or genetic marker with a specific allele, or genetic marker at a nearby chromosomal location more frequently than expected by chance for any particular allele frequency in the population. For example, if locus X has alleles a and b, which occur equally frequently, and linked locus Y has alleles c and d, which occur equally frequently, one would expect the haplotype ac to occur with a frequency of 0.25 in a population of individuals. If ac occurs more frequently, then alleles a and c are considered in linkage disequilibrium. Linkage disequilibrium may result from natural selection of certain combination of alleles or because an allele has been introduced into a population too recently to have reached equilibrium (random association) with between linked alleles.

An "haplotype" is a combination of alleles at multiple loci that are transmitted together on the same chromosome. Haplotype may refer to as few as one locus or to an entire chromosome depending on the number of recombination events that have occurred between a given set of loci.

The term "analysis of the genetic predisposition" encompasses the determination of the probability of an individual to develop an specific phenotype due to his inherent genetic charge. In the case of the invention the term defines the determination of the probability to develop adverse side effects related to bioimplants due to the genetic dotation of each individual.

A "genetic marker" or "marker" is a gene or DNA sequence with a known location on a chromosome that can be used to identify cells, individual or species. It can be described as a variation, which may arise due to mutation or alteration in the genomic loci, that can be observed. A genetic marker may be a short DNA sequence, such as a sequence surrounding a single base-pair change (single nucleotide polymorphism, SNP), or a long one, like minisatellites, translocations, or base pair repetitions.

Terms "bioimplant", "non-metallic bioimplant" and "non-metallic material implanted into the body" are used as synonymous in this description. An implant can be classified as being a metallic implant (e.g.: titanium femur), a ceramic implant (e.g.: hydroxyapatite), a polymeric implant (e.g.: polyalkylimide) or a biological implant (e.g.: botulinum toxin). In this description when non-metallic implants are mentioned it is intended to embrace the ceramic implants, the polymeric material implants or the biological implants. Ceramic are considered inorganic non-metallic materials. When "polymeric material" is used, it is encompassed all natural or biological polymeric materials, such as collagen; as well as the synthetic polymeric materials, such as the polyalkylimide implants. The implants can be applied to a broad area to replace or act as a large portion of tissue, such as a breast silicone implant. Other implants are applied to a narrower area. When applied to narrow areas, most of said implants are also referred as fillers, also known as dermal or sub-dermal fillers. Examples of fillers are collagen, polylactic-L-acid, polyacrylamide, polyalkylimide, hyaluronic acid, polytetrafluoroethylene and methacrylate. Another broadly used filler is calcium hidroxyapatite.

Common areas of application of the non-metallic implants (bioimplants) include orthopedic (especially maxillofacial) re-constructive prosthesis, augmentation breast prosthesis, cardiac prostheses (artificial heart valves like the Chitra heart valve), skin and cornea.

As above indicated, the method of the invention comprises determining whether a biological sample from an individual contains at least the HLA-B*08 antigen of the major histocompatibility complex, wherein the presence of at least said antigen is indicative of genetic predisposition to develop adverse effects.

The determination of the presence of the antigens may be carried out using several techniques. Thus, one way to determine if the antigens are present in a sample is by immunoassay with specific antibodies against the epitopes of the antigens (proteins) HLA-B*08 and HLA-DRB1*03. Other technologies, which will be broadly disclosed below, are based on the detection of the DNA or RNA codifying for said antigens, that is, by detecting the presence of the alleles codifying HLA-B*08 or for HLA-B*08 and HLA-DRB1*03, if any. The presence of the alleles of interest may be carried out using genomic or codifying DNA, which is amplified and then detected by specific probes.In general terms the techniques include the isolation of the genomic or transcript contents, mainly the DNA of the cells from a sample, the subsequent amplification of the locus or loci of interest which is/are further detected by means of specific probes. The locus or loci are finally identified and correlated with a pattern. Depending on the primers pool and the probes the DNA methodologies are classified as low resolution methodologies or high resolution methodologies. High resolution methodologies give information regarding the specific allele of a group of alleles. That is, they inform about the specific sequence or allele subtype, such as HLA-DRB1*0322, HLA-DRB1*0323, HLA-B*0838, HLA-B*0839. Low resolution methodologies allow determining if in a sample an allele of HLA-B*08 and/or the antigen HLA-DRB1*03 is/are present.

Thus, in a preferred embodiment, the method comprises determining the presence of the antigens HLA-B*08 and HLA-DRB1*03; and wherein the presence of the two antigens is indicative of genetic predisposition to develop said adverse effects.

In an embodiment of the method, determining the presence of the antigen HLA-B*08 or of the antigens HLA-B*08 and HLA-DRB1*03 is carried out by genotyping the allele of HLA-B*08 or the alleles of HLA-B*08 and HLA-DRB1*03; or any alteration in linkage disequilibrium with the genes codifying said antigens.

Genotyping the allele is determining the DNA sequence specific for it. The detection of at least one allele codifying for the HLA-B*08 antigen of the major histocompatibility complex is indicative of genetic predisposition to develop adverse effects related to non-metallic materials implanted into the body.

Thus, in an embodiment the method comprises genotyping to determine whether a biological sample from said individual contains simultaneously at least one allele of the antigen HLA-B*08 and at least one allele of the antigen HLA-DRB1*03 of the major histocompatibility complex, defining then an haplotype which is being indicative of genetic predisposition to develop adverse effects related to non-metallic materials implanted into the body.

The individual is preferably a mammal and more preferably a human. Nonetheless, the HLA system or in general terms the human Major Histocompatibility Complex (MHC), has its equivalent set of genes in most vertebrates. Therefore, those genetic sequences between different species that share a high percentage of homology, are probably leading to the same observed effects.

In a more preferred embodiment, the method comprises determining the presence of the allele of the antigen HLA-B*08 selected from the group consisting of: HLA-B*080101 (HLA00146), HLA-B*080102 (HLA02219), HLA-B*080103 (HLA02400), HLA-B*080104 (HLA02853), HLA-B*080105 (HLA03034), HLA-B*080106 (HLA03449), HLA-B*080107 (HLA03939), HLA-B*080108 (HLA04210), HLA-B*080109 (HLA04221), HLA-B*080110 (HLA04504), HLA-B*0802 (HLA00147), HLA-B*0803 (HLA00148), HLA-B*0804 (HLA00149), HLA-B*0805 (HLA00150), HLA-B*0806 (HLA00151), HLA-B*0807 (HLA00974), HLA-B*0808N (HLA00975), HLA-B*0809 (HLA00976), HLA-B*0810 (HLA01082), HLA-B*0811 (HLA01193), HLA-B*081201 (HLA01230), HLA-B*081202 (HLA03935), HLA-B*081203 (HLA04186), HLA-B*0813 (HLA01352), HLA-B*0814 (HLA01418), HLA-B*0815 (HLA01535), HLA-B*0816 (HLA01641), HLA-B*0817 (HLA01654), HLA-B*0818 (HLA01701), HLA-B*0819N (HLA01717), HLA-B*0820 (HLA01752), HLA-B*0821 (HLA01772), HLA-B*0822 (HLA01917), HLA-B*0823 (HLA02132), HLA-B*0824 (HLA02142), HLA-B*0825 (HLA02252), HLA-B*0826 (HLA02308), HLA-B*0827 (HLA02406), HLA-B*0828 (HLA02490), HLA-B*0829 (HLA02496), HLA-B*0830N (HLA02591), HLA-B*0831 (HLA02762), HLA-B*0832 (HLA02771), HLA-B*0833 (HLA 02849), HLA-B*0834 (HLA03091), HLA-B*0835 (HLA03164), HLA-B*0836 (HLA03265), HLA-B*0837 (HLA03450), HLA-B*0838 (HLA03481), HLA-B*0839 (HLA03663), HLA-B*0840 (HLA03648), HLA-B*0841 (HLA03902), HLA-B*0842 (HLA03941), HLA-B*0843 (HLA04081), HLA-B*0844 (HLA04188), HLA-B*0845 (HLA04230), HLA-B*0846 (HLA04234), HLA-B*0847 (HLA04240), HLA-B*0848 (HLA04438), HLA-B*0849 (HLA04442), HLA-B*0850 (HLA04499), HLA-B*0851 (HLA04507), HLA-B*0852 (HLA04515), HLA-B*0853 (HLA04516), HLA-B*0854 (HLA04520), HLA-B*0855 (HLA04708), HLA-B*0856 (HLA04720), HLA- B*0857 (HLA04722), HLA- B*0858 (HLA04753), HLA- B*0859 (HLA04788), HLA- B*0860 (HLA04816), HLA-B*0861 (HLA04860).

In another preferred embodiment, when the presence of both the antigen HLA-B*08 and HLA-DRB1*03 is determined, the method comprises determining the presence of the allele of the antigen HLA-DRB1*03 selected from the group consisting of: HLA-DRB1*03010101 (HLA00671), HLA-DRB1*03010102 (HLA03483), HLA-DRB1*030102 (HLA00672), HLA-DRB1*030103 (HLA00672), HLA-DRB1*030104 (HLA02830), HLA-DRB1*030105 (HLA02955), HLA-DRB1*030106 (HLA03061), HLA-DRB1*030107 (HLA03855), HLA-DRB1*030108 (HLA04577), HLA-DRB1*030109(HLA04691), HLA-DRB1*030201 (HLA00673), HLA-DRB1*030202 (HLA00674), HLA-DRB1*0303 (HLA00675), HLA-DRB1*0304 (HLA00676), HLA-DRB1*030501 (HLA00677), HLA-DRB1*030502 (HLA01428), HLA-DRB1*030503 (HLA03765), HLA-DRB1*0306 (HLA00678), HLA-DRB1*0307 (HLA00679), HLA-DRB1*0308 (HLA00680), HLA-DRB1*0309 (HLA00681), HLA-DRB1*0310 (HLA00682), HLA-DRB1*031101 (HLA00683), HLA-DRB1*031102 (HLA04406), HLA-DRB1*0312 (HLA00684), HLA-DRB1*031301 (HLA01007), HLA-DRB1*031302 (HLA03684), HLA-DRB11*0314 (HLA01008), HLA-DRB1*0315 (HLA01087), HLA-DRB1*0316 (HLA01152), HLA-DRB1*0317 (HLA01153), HLA-DRB1*0318 (HLA01349), HLA-DRB1*0319 (HLA01432), HLA-DRB1*0320 (HLA01455), HLA-DRB1*0321 (HLA01501), HLA-DRB1*0322 8 HLA01557), HLA-DRB1*0323 (HLA01614), HLA-DRB1*0324 (HLA01687), HLA-DRB1*0325 (HLA01692), HLA-DRB1*0326 (HLA01868), HLA-DRB1*0327 (HLA01930), HLA-DRB1*0328 (HLA01933), HLA-DRB1*0329 (HLA02420), HLA-DRB1*0330 (HLA02603), HLA-DRB1*0331 (HLA02606), HLA-DRB1*0332 (HLA02827), HLA-DRB1*0333 (HLA02870), HLA-DRB1*0334 (HLA02886), HLA-DRB1*0335 (HLA02902), HLA-DRB1*0336 (HLA02996), HLA-DRB1*0337 (HLA03059), HLA-DRB1*0338 (HLA03067), HLA-DRB1*0339 (HLA03075), HLA-DRB1*0340 (HLA03370), HLA-DRB1*0341 (HLA03641), HLA-DRB1*0342 (HLA03749), HLA-DRB1*0343 (HLA03836), HLA-DRB1*0344 (HLA03842), HLA-DRB1*0345 (HLA03843), HLA-DRB1*0346 (HLA03844), HLA-DRB1*0347 (HLA03859), HLA-DRB1*0348 (HLA03869), HLA-DRB1*0349 (HLA04355), HLA-DRB1*0350 (HLA04386), HLA-DRB1*0351 (HLA04411), and HLA-DRB1*0352 (HLA04634).

The alleles of the HLA-B*08 and HLA-DRB1*03 above identified are the official sequences (Version of April 1, 2010) as approved by the World Health Organization (WHO) Nomenclature Committee for Factors of the HLA System. The identification into brackets of every allele (HLAxxxxx) refers to the accession number (IMGT/HLA Acc No:) of the sequence defining each allele and listed in he IMGT/HLA Database of the EMBL/GenBank/DDBJ databases.

In a preferred embodiment the genotyping is performed by amplification by primer-specific multiplexed polymerase chain reaction (PCR multiplex) followed by detection.

In a more preferred embodiment, the genotyping is performed by PCR with sequence-specific oligonucleotides (PCR-SSO).

HLA genotyping by polymerase chain reaction (PCR) techniques has become an alternative to serological methods that is widely used in clinical practise. The most commonly used PCR-based typing methods are PCR with sequence-specific primers (PCR-SSP) and PCR with sequence-specific oligonucleotides (PCR-SSO). In the PCR-SSO method, a membrane is prepared (whereon the DNA amplified by the HLA gene specific primers is immobilised) and the specific oligonucleotide probes for the respective type of HLA are hybridised for typing. One example of HLA genotyping by PCR-SSO is the commercial procedure carried out with the Lifecodes HLA-SSO typing kits for use with Luminex® (Lifecodes Molecular Diagnostics).

Most of the technologies used to analyse the genomic contents of a sample employ the DNA of the cells of the individual to be analysed. Nonetheless, RNA can also be used.

Alternatively and as above exposed, the method of the invention may be performed by detecting the presence of the protein codified by the alleles of interest. This kind of detection is mainly done with specific antibodies. The detection with antibodies allows determining if in a sample the antigen HLA-B*08 and/or the antigen HLA-DRB1*03 is/are present, which is already useful for the analysis of the genetic predisposition of an individual to develop adverse effects related to non-metallic materials implanted into the body.

Although the DNA, RNA or proteins to be analysed can be prepared using any of the known methods from any kind of biological sample, a preferred one is a blood sample, from whole blood or cord blood. Other suitable biological samples include blood impregned cards (stain cards), buccal swabs, and urine.

An example of method for the collection and preparation of the sample to be analysed is the QIAAmp® (Qiagen) method.

The method of the invention allows to the analysis of the genetic predisposition of an individual to develop adverse effects related to non-metallic materials implanted into the body, being preferred those broadly used and having an organic chemical structure, such as collagen, polylactic-L-acid, polyacrylamide, polyalkylimide, hyaluronic acid, polytetrafluoroethylene and methacrylate. All these compounds are polymeric material implants. Another polymeric material implant is the silicone oil (or silicone medical grade).

Another material for which the method also provides a suitable analysis of the genetic predisposition of an individual to develop adverse effects, is the inorganic material hydroxyapatite, being preferred the calcium-hydroxyapatite (used broadly in the form of microspheres). Calcium-hydroxyapatite is a kind of ceramic implant.

A mixture of at least two of the above-mentioned non-metallic materials may also be employed for being implanted into the body. The invention provides also with a method to analyse the genetic predisposition to develop adverse effects when more than one of the materials are implanted.

Although several adverse side effects seem to occur when a bioimplant is applied to certain individuals, the method of the invention is specially useful to avoid (due to an analysis "in advance") the so called late-onset adverse effects, which in some cases are severe side effects with a chronic development. Therefore, the method of the invention allows detecting in advance (before implantation) the susceptibility of an individual to present adverse side effects, and thus, it results of great interest. Moreover, the method fills a gap of the state of the art, and at the same time allows to minimize putative cases that had developed not immediately, but perhaps later and slowly to severe and critical pathological conditions.

Some of the adverse effects related to non-metallic materials implanted into the body are selected from the group consisting of implant rejection, lipoatrophy, granulomatous disorders, nodule inflammation, induration, angioedema, sarcoidosis, systemic inflammatory immune-mediated disorders, cutaneous sarcoidosis, pneumonitis, human adjuvant disease and more than one of said adverse effects.

Some of the adverse effects related to non-metallic materials implanted into the body are preferably selected from implant rejection; lipoatrophy related to high intense antiretroviral therapy that include anti-protease drugs (such as the antiretroviral therapy administered to AIDS patients); delayed granulomatous reactions, including granulomas derived from different causes; sarcoidosis; nodulosis; skin induration; abscesses and pseudo-abscesses; fibrosis; angioedema; hypersensitivity; pneumonitis; hepatitis; scleroderma; or inflammatory idiophatic myopathy, most of them framed as immune-mediated disorders. All these adverse effects are correlated with the determination in a sample of the haplotype defined by the presence of at least one allele of the antigen HLA-B*08 /or at least one allele of the antigen HLA-B*08 and at least one allele of the antigen HLA-DRB1*03

IThe antigen HLA-B*08 isemployed as marker for the analysis of the genetic predisposition of an individual to develop adverse effects related to non-metallic materials implanted into the body.

Also the antigens HLA-B*08 and HLA-DRB1*03, are used as markers for the analysis of the genetic predisposition of an individual to develop adverse effects related to non-metallic bioimplants implanted into the body

As above exposed determining the marker is preferably performed by genotyping and determining the haplotype defined by the presence of at least one allele of HLA-B*08 and the presence of at least one allele of HLA-DRB1*03.

When adequate means for carrying out the method of the invention are provided together, a kit is obtained which is useful for the analysis of the genetic predisposition of an individual to develop adverse effects related to materials implanted into the body. The kit can comprise means for carrying out a PCR with sequence-specific oligonucleotides (PCR-SSO), thus including the primers for specific amplification of alleles codifying for the HLA-B*08 and HLA-DRB1*03 to obtain the corresponding amplicons (amplified DNA regions); probes for the isolation and detection of the amplicons; and means for interpreting the results obtained, such as a pattern.

In the same way, the method of the invention is carried out using any other known kit which comprises adequate means for determining the presence of at least one allele of HLA-B*08 /or at least one allele of HLA-B*08 and one allele of HLA-DRB1*03. These kits comprise means for genotyping the allele of HLA-B*08 or the allele of HLA-B*08 and the allele of HLA-DRB1*03, or any alteration in linkage disequilibrium with the genes codifying for said antigens.

Alternatively, the kit used in the method of the invention can comprise adequate means for determining the presence of the proteins, that is, the HLA-B*08 antigen or the HLA-B*08 antigen and the HLA-DRB1*03 antigen. Adequate means include any specific antibody for the antigens and compounds (secondary antibodies, fluorescent or radioisotopic markers, etc) allowing for the detection of the antibody-antigen complexes.

Throughout the description and claims the word "comprise" and variations of the word, are not intended to exclude other technical features, additives, components, or steps. The following examples are provided by way of illustration, and they are not intended to be limiting of the present invention. Furthermore, the present invention covers all possible combinations of particular and preferred embodiments described herein.

### EXAMPLES

This invention is based in part on the discovery and characterization of a novel susceptibility locus for the predisposition to develop adverse side effects after the implantation of non-metallic materials. Said locus is the one of HLA-B*08, or the haplotype HLA-B*08 and HLA-DRB1*03, as well as the proteins (serotypes) derived from the genes of said loci.

### Example A) Sample preparation and results of analysed population (n=245)

Low resolution genotyping of HLA-B and DRB1 alleles of 245 patients and controls was performed by sequence-specific oligonucleotide probe (SSOP) methods using the Luminex microbead technology (Lifecodes HLA-SSO typing kits for use with Luminex®, reference and version LC7751VD.11(02/09) from Tepnel Lifecodes Molecular Diagnostics - Stamford). As it will be illustrated in the Table 1 bellow, some individuals had adverse effects derived from the injection of a bioimplant, such as a dermal or sub-dermal filler of organic or inorganic chemical nature.

The LIFECODES HLA-SSO Typing procedure is based on hybridizing a labeled single stranded PCR amplicon to SSO probes. The probes may be homologous to a sequence within the amplified DNA that is unique for an allele, or to a group of alleles. The probes hybridize to a complementary region that may or may not be present in the amplicon. Consensus probes homologous to sequences present in all the alleles of a locus are also employed, and act as indicators of the success of the amplification and hybridization steps. In the LIFECODES HLA-SSO Typing procedure, the probes are attached to Luminex Microspheres® adapted to be used in the Luminex Instrument®. Several population sof Luminex Microspheres are mixed and analysed together, since each population of microspheres is distinguished by a unique color. This technology is advantageous because it can also be used to quantify the relative amounts of PCR products, thus leading to the determination of the HLA phenotype of every sample (individual).

### A.1.DNA extraction:

DNA was extracted from blood samples of the patients and controls; and it was prepared using the QIAAmp® (Qiagen, S.A.) method. The isolated DNA was preserved in 10 mM TRIS, ph 8.0-9.0 in nuclease-free water. The final concentration of DNA was comprised between 10-200 ng/µl.

### A.2. DNA amplification (PCR):

The reaction components and quantities for the DNA amplification was the following:
- Primer mix (LIFECODES Master Mix); 15 µl
- Genomic DNA; up to 200 ng
- Recombinant Taq polymerase; 0.5 µl (2.5 U)
- Nuclease-free water (Tepnel Lifecodes); to 50 µl of final volume.

The primer mix includes the buffer and the dNTP (deoxynucleotides) to perform the amplification.

The thermal cycler conditions for amplification were the following:
- 5 minutes at 95 °C,
- 1 cycle with the following steps: 95 °C for 30 seconds (s); 60 °C for 45 s; 72 °C for 45 s,
- 8 cycles with the following steps: 95 °C for 30 seconds (s); 63 °C for 45 s; 72 °C for 45 s, and
- 32 cycles at 72 °C for 15 minutes (min).

The amplification was carried out in a Thermal cycler (PCR) 96 well plates (Costar®, No. 6509).

### A.3. Probe hybridization and sample analysis.

An aliquot of 5 µl of every locus specific PCR product was added to a thermal cycler 96 well plate (Costar®, No. 6509), together with an aliquot of 15 µl of probes mix (LIFECODES HLA Probe Mix). The plate was sealed with Microseal® film. The samples were hybridized following the incubation conditions below:
- 97°C for 5 min; 47°C for 30 min; 56°C for 10 min.

Once the hybridization was concluded, 170 µl of a solution of 1:200 Dilution Solution of Streptavidin-Phycoerythrin was added to each well. The samples were then transferred to a Luminex Instrument including the software needed to interpret the results, that is, the probe hit pattern of every sample was compared with the Probe Hit Table provided with the kit.

The following Table 1 illustrates the genotyping of every sample analysed as above exposed, and correlates every haplotype with the presence of adverse effects related to the application of bioimplants. The column "bioimplant" relates to the bioimplant or bioimplant mixture applied to every sample. Column "Observations and/or Adverse effect" relates to the pathological condition observed and to some medical annotations. Samples were individuals to which a non-metallic material was implanted. "Y" means yes; "N" neans NO; "NI" means nodule inflammation; HLA-B* (1) is the allele of HLA-B type inherited from progenitor (1); HLA-B* (2) is the allele of HLA-B type inherited from progenitor (2); HLA-DRB1* (1) is the allele of HLA-DRB1* type inherited from progenitor (1); HLA-DRB1* (2) is the allele of HLA-DRB1 type inherited from progenitor (2)

**Table 1: Correlation of the two alleles of HLA-B and HLA-DRB1 of each individual with the onset of adverse effects.**

| 21**SAMPLE** | **HLA-B* (1)** | **HLA-B* (2)** | **HLA-DRB1* (1)** | **HLA-DRB1* (2)** | **Presence of Adverse Effect** | **Observations and/or Adverse effect.** | **Bioimplant** |
|---|---|---|---|---|---|---|---|
| 197 | 07 | 50 | 03 | 07 | N | | Silicone / Restylane® (Hyaluronic acid) / Gold fibers |
| 31 | 07 | 35 | 04 | 15 | N | | Silicone |
| 174 | 07 | 44 | 04 | 07 | N | | Restylane® (Hyaluronic acid) |
| 205 | 07 | 52 | 07 | 15 | N | | Silicone |
| 206 | 07 | 52 | 07 | 15 | N | | Silicone |
| 146 | 07 | 18 | 11 | 15 | N | | Hyaluronic acid |
| 109 | 08 | 14 | 01 | | N | | Silicone |
| 57 | 08 | 51 | 03 | 04 | N | | Hyaluronic acid |
| 147 | 08 | 40 | 03 | 04 | N | | Aquamid® (polyacrylamide gel) / Radiesse® (Calcium hydroxiapatite) |
| 38 | 08 | 15 | 04 | 11 | N | | Silicone/ New Fill® (Polylactic acid) |
| 172 | 08 | 44 | 07 | | N | | Hyaluronic acid |
| 186 | 08 | 14 | 13 | 16 | N | | Hyaluronic acid |
| 44 | 13 | 44 | 01 | 13 | N | | Silicone / Gold fibers / Happy lift® (Polydioxanone-Polylactic-Caprolactone-X copolymer) |
| 170 | 15 | 35 | 01 | | N | | Silicone |
| 204 | 15 | | 01 | 13 | N | | Goretex® (PTFE-polytetrafluoroethylene) / Silicone /Aquamid® (polyacrylamide gel) / Hyaluronic acid |
| 226 | 15 | 44 | 03 | 04 | N | | Aquamid® (polyacrylamide gel) |
| 62 | 15 | 40 | 04 | 13 | N | | Restylane® (Hyaluronic acid) |
| 21 | 15 | 45 | 04 | 12 | N | | Silicone |
| 33 | 15 | 49 | 11 | 13 | N | | Silicone / Restylane® (Hyaluronic acid) |
| 149 | 18 | 35 | 01 | 11 | N | | Aquamid® (polyacrylamide gel) /Radiesse® (Calcium hydroxiapatite) |
| 181 | 18 | 53 | 03 | 13 | N | | Bioalcamid® (polyalkylimide) |
| 233 | 18 | 49 | 03 | 11 | N | | Hyaluronic acid |
| 150 | 18 | 49 | 11 | | N | | Collagen / Dermalive® (Hyaluronic acid, Acrylic hydrogel) / Hylaform® (Hyaluronic acid) Bioalcamid® (polyalkylimide) / Sculptra® (Poly-L-lactic acid) |
| 138 | 27 | 44 | 01 | 15 | N | | Bioalcamid® (polyalkylimide) / Aquamid® (polyacrylamide gel) |
| 25 | 35 | 51 | 01 | 12 | N | | Silicone |
| 202 | 35 | 44 | 03 | 13 | N | | Silicone / Restylane® (Hyaluronic acid) |
| 23 | 35 | 51 | 03 | 07 | N | | Silicone |
| 65 | 35 | 58 | 04 | 13 | N | | Silicone |
| 232 | 35 | 41 | 04 | 13 | N | | Aquamid® (polyacrylamide gel) |
| 37 | 35 | 44 | 07 | 11 | N | | Silicone / Gold fibers |
| 143 | 35 | 51 | 07 | | N | | Aquamid® (polyacrylamide gel) |
| 180 | 35 | 44 | 11 | | N | | Silicone |
| 159 | 38 | 45 | 01 | 04 | N | | Silicone |
| 139 | 38 | 51 | 11 | 13 | N | | Aquamid® (polyacrylamide gel) |
| 58 | 39 | 41 | 13 | 14 | N | | Bioalcamid® (polyalkylimide) |
| 179 | 40 | 44 | 07 | 11 | N | | Restylane® (Hyaluronic acid) |
| 105 | 44 | 56 | 01 | 07 | N | | Achyal® (Hyaluronic acid) / Silicone / Restylane® (Hyaluronic acid) |
| 24 | 44 | 50 | 01 | 11 | N | | Silicone |
| 127 | 44 | | 07 | 09 | N | | Restylane® (Hyaluronic acid) / Achyal® (Hyaluronic acid) |
| 160 | 44 | | 07 | | N | | Silicone |
| 196 | 45 | | 07 | 15 | N | | Silicone |
| 79 | 49 | 51 | 04 | 10 | N | | Collagen / Dermalive® (Hyaluronic acid, Methacrylate) / New Fill® (Polylactic acid) / Hyaluronic acid |
| 98 | 49 | 50 | 04 | 15 | N | | Restylane® (Hyaluronic acid) |
| 141 | 49 | 50 | 13 | | N | | Aquamid® (polyacrylamide gel) |
| 207 | 51 | 67 | 01 | 03 | N | | Radiesse® (Calcium hydroxiapatite) |
| 194 | 07 | 14 | 01 | 15 | Y | granulomas | Polylactic acid |
| 236 | 07 | 53 | 01 | 15 | Y | | Silicone |
| 134 | 07 | 44 | 04 | 11 | Y | Low reactivity | Bioalcamid® (polyalkylimide) |
| 135 | 08 | 37 | 01 | 14 | Y | nodule inflammation (NI). | Hyaluronic acid |
| 241 | 08 | 13 | 01 | 03 | Y | granulomas | Aquamid® (polyacrylamide gel) |
| 4 | 08 | 39 | 03 | | Y | vasculitis | Restylane® (Hyaluronic acid) |
| 10 | 08 | 44 | 03 | 15 | Y | residual activity/nodule inflammation | Dermalive® (Hyaluronic acid, Methacrylatel) |
| 157 | 08 | 57 | 03 | 07 | Y | nodule inflammation | Restylane® (Hyaluronic acid) |
| 168 | 08 | 44 | 03 | 15 | Y | nodule inflammation/cutaneous induration | Artecoll® (polymethylmethacrylate, collagen) / Silicone |
| 171 | 08 | 44 | 03 | 07 | Y | active granuloma | Bioalcamid® (polyalkylimide) |
| 220 | 08 | 14 | 03 | | Y | severe granulomas although corticoid tretament is followed | Bioalcamid® (polyalkylimide) |
| 1 | 08 | 14 | 03 | 15 | Y | siccus syndrome/granulomas/angioedema | Bioalcamid® (polyalkylimide) |
| 223 | 08 | 27 | 03 | 08 | Y | NI/pseudoabsceses | Aquamid® (polyacrylamide gel) |
| 240 | 08 | 14 | 03 | 07 | Y | NI (light) | Aquamid® (polyacrylamide gel) |
| 238 | 13 | 35 | 04 | 13 | Y | granulomas although corticoid tretament is followed | Bioalcamid® (polyalkylimide) |
| 234 | 13 | 44 | 07 | | Y | induration/angioedema | Silicone |
| 13 | 13 | 49 | 13 | 16 | Y | no reactivity against silicone 12 years before | Dermalive® (Hyaluronic acid, Acrylic hydrogel) |
| 11 | 14 | 44 | 07 | 12 | Y | | Bioalcamid® (polyalkylimide)/collagen |
| 235 | 15 | 44 | 01 | 09 | Y | | Silicone |
| 239 | 15 | 44 | 04 | 11 | Y | | Silicone |
| 163 | 15 | 51 | 07 | 11 | Y | NI | Silicone |
| 208 | 15 | 18 | 11 | 15 | Y | acute face reaction after second implantation. | Sculptra® (Poly-L-lactic acid) |
| 222 | 15 | 41 | 13 | | Y | granulomas/induration | Hyaluronic acid |
| 101 | 18 | | 03 | | Y | granulomas/induration | Bioalcamid® (polyalkylimide) |
| 209 | 18 | 40 | 03 | 04 | Y | surgical extirpation needed | Dermalive® (Hyaluronic acid, Methacrylatel) |
| 244 | 18 | 51 | 03 | 04 | Y | surgical extirpation needed | Bioalcamid® (polyalkylimide) |
| 111 | 18 | 52 | 11 | 13 | Y | | Silicone |
| 68 | 35 | 51 | 01 | 04 | Y | recurrent sarcoidosis (remittent) | Dermalive® (Hyaluronic acid, Methacrylate)/ Polylactic |
| 41 | 35 | 39 | 04 | | Y | acute reaction. | Silicone |
| 169 | 35 | 39 | 04 | | Y | fluctuant activity | Silicone |
| 5 | 35 | 44 | 07 | | Y | residual activity | Bioalcamid® (polyalkylimide) |
| 219 | 35 | 44 | 07 | 09 | Y | NI | Aquamid® (polyacrylamide gel) |
| 162 | 35 | 44 | 13 | | Y | NI | Silicone |
| 2 | 38 | 44 | 11 | 13 | Y | receding | Dermalive® (Hyaluronic acid, Methacrylate) |
| 158 | 38 | 44 | 11 | 13 | Y | NI and pruritis | Silicone |
| 242 | 38 | 39 | 14 | | Y | | Silicone |
| 28 | 39 | 40 | 03 | 16 | Y | receding granuloma | Dermalive® (Hyaluronic acid, Methacrylate) |
| 43 | 40 | 44 | 07 | 12 | Y | silent granuloma | Bioalcamid® (polyalkylimide) |
| 136 | 40 | 44 | 11 | 14 | Y | | Outline Ultra® (Hyaluronic acid) |
| 137 | 44 | | 01 | 07 | Y | | Silicone |
| 100 | 44 | 52 | 03 | 11 | Y | | Restylane® (Hyaluronic acid) |
| 36 | 44 | 51 | 04 | 07 | Y | granuloma | Bioalcamid® (polyalkylimide) / Aquamid® (polyacrylamide gel) / Evolence® (Collagen) |
| 140 | 44 | 51 | 12 | 14 | Y | non active postraumatic granuloma | Silicone |
| 70 | 44 | 52 | 13 | 14 | Y | NI | Dermalive® (Hyaluronic acid, Methacrylate) |
| 40 | 49 | 55 | 04 | | Y | breast prosthesis 10 years later/ (NI/induration) | Methacrylate |
| 8 | 51 | | 01 | 04 | Y | receding | Silicone |
| 245 | 51 | 57 | 04 | 15 | Y | granulomas | Bioalcamid® (polyalkylimide) |

From Table 1 it is deduced that using the predictive test or method of the invention, late-onset adverse effects and some immediate adverse effects related to bioimplants, namely dermal and sub-dermal fillers, could be prevented in the 30-40 % of the individuals that opted to the implantation of biomaterials, and showed late-onset adverse effects. In other words, the probability of developing an adverse effect is greater in an individual comprising at least one allele of the antigen HLA-B*08.

More concretely, a late-onset adverse effect related to a bioimplant is 600-fold in individuals comprising the haplotype defined by the simultaneous presence of one allele of HLA-B*08 and one allele of HLA-DRB1*03 (haplotype HLA-B*08/ HLA-DRB1*03). The relative risk (rr) when presenting one allele of the antigens *versus* the relative risk when presenting the haplotype HLA-B*08/ HLA-DRB1*03 is 1 to 5.9 (rr=1 vs. rr=5.9).

All the data of Table 1 were statistically analysed using the Fisher's exact test (2 tail) with a confidence interval of 95 %. The samples comprising the haplotype HLA-B*08/ HLA-DRB1*03 revealed a value for odds ratio of 5.81 and the Fisher's exact test was lower than 0.02.

Therefore, although the presence of the haplotype HLA-B*08/ HLA-DRB1*03 implies a greater risk of developing adverse effects related to bioimplants, the presence of at least one single allele of HLA-B*08 has to be considered as a risk factor too, according to the data of Table 1.

From Table 1 it can also be extracted that some late-onset adverse effects may be developed correlating with a different haplotype than that of HLA-B*08 and HLA-DRB1*03. These cases cannot be detected with the method of the invention and further studies are needed.

In a more detailed manner Table 1 shows the adverse side effects detected. Delayed granulomatous reactions have been related to collagen, silicone, polylactic-L-acid, polyacrilamide, polyalkylimide, hyaluronic acid, polytetrafluoroethylene (Gore-Tex®) and methacrylate implants. The same type of adverse reactions have been described with the use of combinations of methacrylate-collagen (Artecoll®) or ethylmethacrylate-hyaluronic (Dermalive®). Some different late adverse reactions to polyalkylimide have also been reported. Even different and specific histological granulomas related to hyaluronic acid (HA-AH, Dermalive®), methacrylate-collagen (Artecoll®), polylactic acid (New-Fill®), silicone and polyacrilamide (Aquamid®) have been described. The bioimplant capacity to induce immune-mediated adverse effects in humans may be related to the different chemical composition of the bioimplants and the biologic characteristicsof the individual. It is known that all bioimplants can, *in vitro,* elicit a primary immune-mediated foreign-body type reaction based on macrophage activation and the possible induction of T-cell response. In theory and regarding to granulomas, gradual development of network collagen fibres around the bioimplant particles appears to coincide with a decrease in the inflammatory reaction to the same. But so-called stable granulomas may evolve into a progressive abnormal granulomatous response to bioimplants, such as dermal or sub-dermal fillers. Sometimes, undesirable effects appear during minor trauma or after infection. It is hypothesised that the own and specific genetic background must play an important role in the development of these adverse reactions.

### Example B) Clinical case.

A 60-years old patient presenting athopic antecedents and a putative drug hypersensitivity, and who wanted to be treated by a surgeon to improve wrinkles of the face, was firstly analysed by means of the method of the present invention. It was intended to determine if the patient was susceptible of implant (sub-dermal filler) rejection or development of adverse-side effects. The filler which had been selected would be hyaluronic acid. After performing a classical analysis (determination of ANAs, CH50, C4, proteinogram..); and after experimentally testing the haplotype of the HLA-B and HLA-DRB1 loci (using the Lifecodes HLA-SSO typing kits for use with Luminex®, LC775IVD.11 (02/09) from Tepnel Lifecodes Molecular Diagnostics - Stamford), it was determined that the patient presented the following haplotype: HLA-B*08 and HLA-DRB1*03. The surgeon refused injecting the hyaluronic acid filler and proceeded only with a face lifting.

### Example C) Other clinical cases.

C1. A 45-years old patient sought medical attention due to the onset of face nodules, cutaneous lesions and sicca syndrome 18 months later the implantation of face implants (filler injection) made of hyaluronic acid and methacrylate. Blood test revealed hypergammaglobulinemia, high levels of acute phase reactants, as well as high values of the angiotensin conversing enzyme. After experimentally testing the haplotype of the HLA-B and HLA-DRB1 loci from buccal swabs, it was determined that the patient presented the following haplotype: HLA-B*08 and HLA-DRB1*03 (using the Lifecodes HLA-SSO typing kits for use with Luminex®, LC775IVD.11 (02/09) from Tepnel Lifecodes Molecular Diagnostics - Stamford). The patient was duly informed about the high risk of developing adverse effects in case of new bioimplants (filler injection) were used.

C2. A patient who was derived from the dermatological specialist in order to determine a putative risk of late-onset adverse effect related to bioimplants was analysed. The patient seemed to have developed a previous reaction on the face after implantation of collagen and methacrylate. After experimentally testing, as done in Example B, the haplotype of the HLA-B and HLA-DRB1 loci from blood, it was determined that the patient presented the following haplotype: HLA-B*08 and HLA-DRB1*03. The patient was duly informed about the high risk of developing adverse effects in case of new bioimplants (fillers) were used. Also the surgeon was informed and desestimated the injection of any filler. However, the patient consulted another specialist who acceded to inject the filler hyaluronic acid. Three months after the injection of the filler a severe inflammation outbreak was observed in the first and second treated zones of the face.

To the best of the inventors' knowledge, by now no significant statistical data have been reported correlating the genetic predisposition of an individual to develop adverse effects related to materials implanted into the body. This can be due to the fact that the firms commercializing the bioimplants have been silent about the risk of side effects derived from the implants; or simply because most of the adverse effects are late-onset adverse effects, difficult to be immediately correlate with the implantation of a biomaterial some years ago.

Emphasis is made to the fact that in all the tested individuals (patients or controls), no reactive antibodies against the bioimplant were detected. Thus, the inflammatory reactions and immune disorders observed are not due to an autoimmune reaction pattern. In the same way, specific auto-antibodies directed to different bioimplants, such as fillers, have not been detected in the samples currently. Additionally, the biopsies observed in case of adverse side effects related to bioimplants are quite different from those observed in autoimmune diseases. Usually, the histophatological pattern observed in these biopsies is characteristic of type IV hypersensitivity reaction (Gell and Coombs classification). Thus, the T-cell more than B-cell involvement is demonstrated. Nonetheless, the evolution of inflammation to a chronic state could evolve to the development or "wakening up" of autoimmune disorders, such as vasculitis, polymyositis, Sjögren syndrome, etc. These severe disorders can be avoided when the method of the invention is performed in an individual who was suggested to receive a bioimplant (non-metallic material implant).

The method of the invention results from the first significantly statistical assay that correlates an haplotype or the presence of some alleles of the HLA system, or the presence of the proteins (antigens) codified by the alleles, with the risk of developing adverse effects after the implantation of a biomaterial. Although and fortunately the prevalence of the adverse effects related to the bioimplants is low, the onset of said adverse effects can be really severe. Thus, the method of the invention is important because it allows the discrimination of a surgical or cosmetic treatment that could lead to the development of severe adverse effects, contrarty to the aim essence of the cosmetic treatment.

The present invention provides for the first time significant statistical data associating a specific haplotype with the susceptibility or predisposition of an individual to develop (late-onset or immediate) adverse effects derived from materials implanted into the body, namely dermal and sub-dermal fillers.

### REFERENCES CITED IN THE APPLICATION

- Di Lorenzo et al., "Morphea after Silicone Gel Breast Implantation for Cosmetic Reasons in an HLA-BS, DR3-Positive Woman, Int. Arch Allergy Immunol 1997, vol. 112, pp. 93-95.

## Claims

1. An *in vitro* method for the analysis of the genetic predisposition of an individual to develop adverse effects related to non-metallic bioimplants implanted into the body, comprising determining, in a biological sample from said individual, the presence of the antigen HLA-B*08 of the major histocompatibility complex;
and wherein the presence of at least said antigen is indicative of genetic predisposition to develop said adverse effects.

2. The method according to claim 1, which comprises determining the presence of the antigens HLA-B*08 and HLA-DRB1*03; and wherein the presence of the two antigens is indicative of genetic predisposition to develop said adverse effects.

3. The method according to any of the claims 1-2, wherein determining the presence of the antigen HLA-B*08 or of the antigens HLA-B*08 and HLA-DRB1*03 is carried out by genotyping the allele of HLA-B*08 or the alleles of HLA-B*08 and HLA-DRB1*03; or any alteration in linkage disequilibrium with the genes codifying said antigens.

4. The method according to claim 3, wherein the allele of the antigen HLA-B*08 is selected from the group consisting of: HLA-B*080101, HLA-B*080102, HLA-B*080103, HLA-B*080104, HLA-B*080105, HLA-B*080106 , HLA-B*080107, HLA-B*080108 , HLA-B*080109, HLA-B*080110, HLA-B*0802, HLA-B*0803, HLA-B*0804 , HLA-B*0805, HLA-B*0806 , HLA-B*0807, HLA-B*0808N, HLA-B*0809, HLA-B*0810, HLA-B*0811, , HLA-B*081201, HLA-B*081202 , HLA-B*081203, HLA-B*0813, HLA-B*0814, HLA-B*0815, HLA-B*0816 , HLA-B*0817, HLA-B*0818, HLA-B*0819N, HLA-B*0820, HLA-B*0821 HLA-B*0822, HLA-B*0823, HLA-B*0824, HLA-B*0825, HLA-B*0826, HLA-B*0827, HLA-B*0828, HLA-B*0829, HLA-B*0830N, HLA-B*0831, HLA-B*0832, HLA-B*0833, HLA-B*0834, HLA-B*0835, HLA-B*0836, HLA-B*0837, HLA-B*0838, HLA-B*0839, HLA-B*0840, HLA-B*0841, HLA-B*0842, HLA-B*0843, HLA-B*0844, HLA-B*0845, HLA-B*0846, HLA-B*0847, HLA-B*0848, HLA-B*0849, HLA-B*0850, HLA-B*0851, HLA-B*0852, HLA-B*0853, HLA-B*0854, HLA-B*0855, HLA-B*0856, HLA-B*0857, HLA- B*0858, HLA- B*0859, HLA- B*0860, and HLA-B*0861.

5. The method according to claim 3, wherein the allele of the antigen HLA-DRB1*03 is selected from the group consisting of: HLA-DRB1*03010101, HLA-DRB1*03010102, HLA-DRB1*030102 , HLA-DRB1*030103, HLA-DRB1*030104, HLA-DRB1*030105, HLA-DRB1*030106, HLA-DRB1*030107, HLA-DRB1*030108, HLA-DRB1*030201, HLA-DRB1*030202, HLA-DRB1*0303, HLA-DRB1*0304, HLA-DRB1*030501, HLA-DRB1*030502, HLA-DRB1*030503, HLA-DRB1*0306, HLA-DRB1*0307, HLA-DRB1*0308, HLA-DRB1*0309, HLA-DRB1*0310, HLA-DRB1*031101, HLA-DRB1*031102, HLA-DRB1*0312, HLA-DRB1*031301, HLA-DRB1*031302, HLA-DRB1*0314, HLA-DRB1*0315, HLA-DRB1*0316 HLA-DRB1*0317, HLA-DRB1*0318, HLA-DRB1*0319, HLA-DRB1*0320, HLA-DRB1*0321, HLA-DRB1*0322, HLA-DRB1*0323, HLA-DRB1*0324, HLA-DRB1*0325, HLA-DRB1*0326, HLA-DRB1*0327, HLA-DRB1*0328, HLA-DRB1*0329, HLA-DRB1*0330, HLA-DRB1*0331, HLA-DRB1*0332, HLA-DRB1*0333, HLA-DRB1*0334, HLA-DRB1*0335, HLA-DRB1*0336, HLA-DRB1*0337, HLA-DRB1*0338, HLA-DRB1*0339, HLA-DRB1*0340, HLA-DRB1*0341, HLA-DRB1*0342, HLA-DRB1*0343, HLA-DRB1*0344, HLA-DRB1*0345, HLA-DRB1*0346, HLA-DRB1*0347, HLA-DRB1*0348, HLA-DRB1*0349, HLA-DRB1*0350, HLA-DRB1*0351, and HLA-DRB1*0352.

6. The method according to any of claims 3-5, wherein the genotyping is performed by amplification by primer-specific PCR multiplex followed by detection.

7. The method according to any of the claims 1-6, wherein the biological sample is a blood sample.

8. The method according to any of the claims 1-7, wherein the non-metallic bioimplant implanted into the body is a polymeric material.

9. The method according to claim 8, wherein the polymeric material is selected from the group consisting of collagen, polylactic-L-acid, polyacrylamide, polyalkylimide, hyaluronic acid, polytetrafluoroethylene, methacrylate, silicone and mixtures thereof.

10. The method according to any of the claims 1-9, wherein the adverse effects related to non-metallic materials implanted into the body are selected from the group consisting of implant rejection, lipoatrophy, granulomatous disorders, nodule inflammation, induration, angioedema, sarcoidosis, systemic inflammatory immune-mediated disorders, cutaneous sarcoidosis, pneumonitis, human adjuvant disease and more than one of said adverse effects.

11. Use of a kit for carrying out the method as defined in any of claims 1-10, wherein the kit comprises adequate means for determining the presence of the antigen HLA-B*08 or the presence of the antigens HLA-B*08 and HLA-DRB1*03 of the major histocompatibility complex.

12. The use of a kit according to claim 11, wherein the kit comprises adequate means for genotyping the allele of HLA-B*08, or for genotyping the alleles of HLA-B*08 and HLA-DRB1*03, or any alteration in linkage disequilibrium with the genes codifying said antigens.

13. In vitro use of the antigen HLA-B*08, as marker for the analysis of the genetic predisposition of an individual to develop adverse effects related to non-metallic bioimplants implanted into the body.

14. In vitro use of the antigens HLA-B*08 and HLA-DRB1*03, as markers for the analysis of the genetic predisposition of an individual to develop adverse effects related to non-metallic bioimplants implanted into the body.

## Patentansprüche

1. Ein In-vitro-Verfahren für die Analyse der genetischen Prädisposition eines Menschen zur Entwicklung von ungünstigen Auswirkungen im Zusammenhang mit nicht-metallischen im Körper implantierten Bioimplantaten umfassend das Ermitteln, in einer biologischer Probe des Menschen, der Anwesenheit des Antigens HLA-B*08 des Haupthistokompatibilitätskomplexes;
und wobei die Anwesenheit von mindesten dem Antigen hinweisend auf die genetische Prädisposition zur Entwicklung von den ungünstigen Auswirkungen ist.

2. Das Verfahren nach Anspruch 1, welches das Ermitteln der Anwesenheit der Antigene HLA-B*08 und HLA-DRB1*03 umfasst; und wobei die Anwesenheit der zwei Antigene hinweisend auf die genetische Prädisposition zur Entwicklung von diesen ungünstigen Auswirkungen ist.

3. Das Verfahren nach einem der Ansprüche 1-2, wobei das Ermitteln der Anwesenheit des Antigens HLA-B*08 oder der Antigene HLA-B*08 und HLA-DRB1*03 indem ausgeführt wird, dass das Allel des HLA-B*08 oder die Allele der HLA-B*08 und HLA-DRB1*03, oder jede Veränderung des Kopplungsungleichgewichts mit den Genen, die die Antigene kodieren, genotypisiert werden.

4. Das Verfahren nach Anspruch 3, wobei das Allel des Antigens HLA- B*080 ausgewählt ist aus der Gruppe bestehend aus: HLA-B*080101, HLA-B*080102, HLA-B*080103, HLA-B*080104, HLA-B*0801 05, HLA-B*080106, HLA-B*080107, HLA-B*080108, HLA-B*080109, HLA-B*080110, HLA-B*0802, HLA-B*0803, HLA-B*0804, HLA-B*0805, HLA-B*0806, HLA-B*0807, HLA-B*0808N, HLA8*0809, HLA-B*0810, HLA-B*0811, HLA-B*081201, HLA-B*081202, HLA-B*081203, HLA-B*0813, HLA-B*0814, HLA-B*0815, HLA-B*0816, HLA-B*0817, HLA-B*0818, HLA-B*0819N, HLA-B*0820, HLA-B*0821, HLA-B*0822, HLA-B*0823, HLA-B*0824, HLA-B*0825, HLA B*0826, HLA-B*0827, HLA-B*0828, HLA-B*0829, HLA-B*0830N, HLA-B*0831, HLA-B*0832, HLA-B*0833, HLA-B*0834, HLA-B*0835, HLA-B*0836, HLA-B*0837, HLA-B*0838, HLA-B*0839, HLA-B*0840, HLA-B*0841, HLA-B*0842, HLA-B*0843, HLA-B*0844, HLA-B*0845, HLA-B*0846, HLA-B*087, HLA-B*0848, HLA-B*0849, HLA-B*0850, HLA-B*0851, HLA-B*0852, HLA-B*0853, HLA-B*0854, HLA-B*0855, HLA-B*0856, HLA- B*0857, HLA-B*0858, HLA- B*0859, HLA- B*0860 und HLA-B*0861.

5. Das Verfahren nach Anspruch 3, wobei das Allel des Antigens HLA-DRB1*03 ausgewählt ist aus der Gruppe bestehend aus: HLA-DRB1*03010101, HLA-DRB1*03010102, HLA-DRB1*030102,HLA-DRB1*030103, HLA-DRB1*030104, HLA-DRB1*030105, HLA-DRB1*030106,HLA-DRB1*030107, HLADRB1*030108, HLA-DRB1*030201, HLADRB1*030202, HLA-DRB1*0303, HLA-DRB1*0304, HLA-DRB1*030501, HLA-DRB1*030502, HLA-DRB1*030503, HLADRB1*0306, HLA-DRB1*0307, HLA-DRB1*0308, HLA-DRB1*0309, HLADRB1*0310, HLA-DRB1*031101, HLA-DRB1*031102, HLA-DRB1*0312, HLADRB1*-031301, HLA-DRB1*031302, HLADRB1*0314, HLA-DRB1*0315, HLA-DRB1*0316, HLA-DRB1*0317, HLADRB1*0318, HLA-DRB1*0319, HLA DRB1*0320, HLA-DRB1*0321, HLA-DRB1*0322, HLA-DRB1*0323, HLA-DRB1*0324, HLA-DRB1*0325, HLA-DRB1*0326, HLA-DRB1*0327, HLA-DRB1*0328, HLA-DRB1*0329, HLA-DRB1*0330, HLA-DRB1*0331, HLA-DRB1*0332, HLA-DRB1*0333, HLA- DRB1*0334, HLA-DRB1*0325, HLA-DRB1*0336, HLA-DRB1*0337, HLA-DRB1*0338, HLA-DRB1*0339, HLA-DRB1*0340, HLA-DRB1*0341, HLA-DRB1*0342, HLA-DRB1*0343, HLA-DRB1*0344, HLA-DRB1*0345, HLA-DRB1*0346, HLA-DRB1*0347, HLA-DRB1*0348, HLA-DRB1*0349, HLA-DRB1*0350, HLA-DRB1*0351, und HLA-DRB1*0352.

6. Das Verfahren nach einem der Ansprüche 3-5, wobei die Genotypisierung durch Primer-spezifische multiplex-PCR-Amplifikation gefolgt von Detektion ausgeführt wird.

7. Das Verfahren nach einem der Ansprüche 1-6, wobei die biologische Probe eine Blutprobe ist.

8. Das Verfahren nach einem der Ansprüche 1-7, wobei das im Körper implantierte nicht-metallische Bioimplantat ein polymeres Material ist.

9. Das Verfahren nach Anspruch 8, wobei das polymere Material ausgewählt aus der Gruppe bestehend aus Kollagen, Polymilch-L-säure, Polyacrylamid, Polyalkylimid, Hyaluronsäure, Polytetrafluoroethylen, Methacrylat, Silicon und Mischungen davon ist.

10. Das Verfahren nach einem der Ansprüche 1-9, wobei die ungünstigen Auswirkungen im Zusammenhang mit im Körper implantierten nicht-metallischen Materialien ausgewählt sind aus der Gruppe bestehend aus Abstoßung von Implantaten, Lipoatrophie, granulomatösen Erkrankungen, Knötchenentzündung, Induration, Angioödem, Sarkoidose, systemischen immunvermittelten Entzündungsstörungen, kutaner Sarkoidose, Pneumonitis, menschlicher Adjuvans-Krankheit und mehr als einer von diesen ungünstigen Auswirkungen.

11. Verwendung eines Kits zur Durchführung des Verfahrens wie in einem der Ansprüche 1-10 definiert, wobei das Kit ein angemessenes Mittel zur Ermittlung der Anwesenheit des Antigens HLA-B*08 oder der Anwesenheit der Antigene HLA-B*08 und HLA-DRB1*03 des Haupthistokompatibilitätskomplexes umfasst.

12. Die Verwendung eines Kits nach Anspruch 11, wobei das Kit ein angemessenes Mittel zur Genotypisierung des Allels von HLA-B*08, oder zur Genotypisierung der Allele der HLA-B*08 und HLA-DRB1*03, oder jeder Veränderung des Kopplungsungleichgewichts mit den Genen, die die Antigene kodieren, umfasst.

13. In-vitro-Verwendung des Antigens HLA-B*08 als Marker für die Analyse der genetischen Prädisposition eines Menschen zur Entwicklung von ungünstigen Auswirkungen im Zusammenhang mit nicht-metallischen im Körper implantierten Bioimplanteten.

14. In-vitro-Verwendung der Antigene HLA-B*08 und HLA-DRB1*03 als Marker für die Analyse der genetischen Prädisposition eines Menschen zur Entwicklung von ungünstigen Auswirkungen im Zusammenhang mit nicht-metallischen im Körper implantierten Biomplantaten.

## Revendications

1. Une méthode *in vitro* pour l'analyse de la prédisposition génétique d'un individu à développer des effets défavorables suite à des implants biologiques non-métalliques implantés dans le corps, comprenant déterminer, dans un échantillon biologique dudit individu, la présence de l'antigène HLA-B*08 du complexe majeur d'histocompatibilité ;
et dans laquelle la présence d'au moins ledit antigène est indicative de la prédisposition génétique à développer lesdits effets défavorables.

2. La méthode selon la revendication 1 qui comprend déterminer la présence des antigènes HLA-B*08 et HLA-DRB1*03; et dans laquelle la présence des deux antigènes est indicative de la prédisposition génétique à développer lesdits effets défavorables.

3. La méthode selon l'une quelconque des revendications 1-2, dans laquelle la détermination de la présence de l'antigène HLA-B*08 ou des antigènes HLA-B*08 et HLA-DRB1*03 est effectuée moyennant le génotypage de l'allèle du HLA-B*08 ou les allèles des HLA-B*08 et HLA-DRB1*03, ou une altération quelconque dans le déséquilibre de liaison avec les gènes qui codifient lesdits antigènes.

4. La méthode selon la revendication 3, dans laquelle l'allèle de l'antigène HLA-B*080 est choisi dans le groupe constitué de : HLA-B*080101, HLA-B*080102, HLA-B*080103, HLA-B*080104, HLA-B*0801 05, HLA-B*080106, HLA-B*080107, HLA-B*080108, HLA-B*080109, HLA-B*080110, HLA-B*0802, HLA-B*0803, HLA-B*0804, HLA-B*0805, HLA-B*0806, HLA-B*0807, HLA-B*0808N, HLA8*0809, HLA-B*0810, HLA-B*0811, HLA-B*081201, HLA-B*081202, HLA-B*081203, HLA-B*0813, HLA-B*0814, HLA-B*0815, HLA-B*0816, HLA-B*0817, HLA-B*0818, HLA-B*0819N, HLA-B*0820, HLA-B*0821, HLA-B*0822, HLA-B*0823, HLA-B*0824, HLA-B*0825, HLA-B*0826, HLA-B*0827, HLA-B*0828, HLA-B*0829, HLA-B*0830N, HLA-B*0831, HLA-B*0832, HLA-B*0833, HLA-B*0834, HLA-B*0835, HLA-B*0836, HLA-B*0837, HLA-B*0838, HLA-B*0839, HLA-B*0840, HLA-B*0841, HLA-B*0842, HLA-B*0843, HLA-B*0844, HLA-B*0845, HLA-B*0846, HLA-B*087, HLA-B*0848, HLA-B*0849, HLA-B*0850, HLA-B*0851, HLA-B*0852, HLA-B*0853, HLA-B*0854, HLA-B*0855, HLA-B*0856, H LA- 8*0857, HLA-B*0858, H LA- 8*0859, H LA- 8*0860 et HLA-B*0861.

5. La méthode selon la revendication 3, dans laquelle l'allèle de l'antigène HLA-DRB1*03 est choisi dans le groupe constitué de : HLA-DRB1*03010101, HLA-DRB1*03010102, HLA-DRB1*030102,HLA-DRB1*030103, HLA-DRB1*030104, HLA-DRB1*030105, HLA-DRB1*030106,HLA-DRB1*030107, HLA-DRB1*030108, HLA-DRB1*030201, HLADRB1*030202, HLA-DRB1*0303, HLA-DRB1*0304, HLA-DRB1*030501, HLA-DRB1*030502, HLA-DRB1*030503, HLADRB1*0306, HLA-DRB1*0307, HLA-DRB1*0308, HLA-DRB1*0309, HLA-DRB1*0310, HLA-DRB1*031101, HLA-DRB1*031102, HLA-DRB1*0312, HLA-DRB1*-031301, HLA-DRB1*031302, HLA-DRB1*0314, HLA-DRB1*0315, HLA-DRB1*0316, HLA-DRB1*0317, HLADRB1*0318, HLA-DRB1*0319, HLA-DRB1*0320, HLA-DRB1*0321, HLA-DRB1*0322, HLA-DRB1*0323, HLA-DRB1*0324, HLA-DRB1*0325, HLA-DRB1*0326, HLA-DRB1*0327, HLA-DRB1*0328, HLA-DRB1*0329, HLA-DRB1*0330, HLA-DRB1*0331, HLA-DRB1*0332, HLA-DRB1*0333, HLA- DRB1*0334, HLA-DRB1*0325, HLA-DRB1*0336, HLA-DRB1*0337, HLA-DRB1*0338, HLA-DRB1*0339, HLA-DRB1*0340, HLA-DRB1*0341, HLA-DRB1*0342, HLA-DRB1*0343, HLA-DRB1*0344, HLA-DRB1*0345, HLA-DRB1*0346, HLA-DRB1*0347, HLA-DRB1*0348, HLA-DRB1*0349, HLA-DRB1*0350, HLA-DRB1*0351, et HLA-DRB1*0352.

6. La méthode selon l'une quelconque des revendications 3-5, dans laquelle le génotypage est effectué par amplification moyennant PCR multiplex spécifique de primer suivie de détection.

7. La méthode selon l'une quelconque des revendications 1-6, dans laquelle l'échantillon biologique est un échantillon de sang.

8. La méthode selon l'une quelconque des revendications 1-7, dans laquelle l'implant biologique non-métallique implanté dans le corps est un matériau polymérique.

9. La méthode selon la revendication 8, dans laquelle le matériau polymérique est choisi dans le groupe constitué du collagène, de l'acide poly-L-lactique, de la polyacrylamide, de la polyacrylimide, de l'acide hyaluronique, du polytétrafluoroéthylène, du méthacrylate, de la silicone et des mélanges de ceux-ci.

10. La méthode selon l'une quelconque des revendications 1-9, dans laquelle les effets défavorables suite à des matériaux non-métalliques implantés dans le corps sont choisis dans le groupe constitué des rejets des implants, de la lipoathrophie, des troubles granulomateuses, de l'inflammation nodulaire, de l'induration, de l' angioedème, de la sarcoïdose, des troubles inflammatoires systémiques à médiation immunitaire, de la sarcoïdose cutanée, de la pneumonite, de la maladie adjuvante chez les humains et plus d'un desdits effets défavorables.

11. Utilisation d'un kit pour effectuer la méthode telle que définie dans l'une quelconque des revendications 1-10, dans laquelle le kit comprend des moyens appropriés pour déterminer la présence de l'antigène HLA-B*08 ou la présence des antigènes HLA-B*08 et HLA-DRB1*03 du complexe majeur d'histocompatibilité.

12. L'utilisation d'un kit selon la revendication 11, dans laquelle le kit comprend des moyens appropriés pour le génotypage de l'allèle de HLA-B*08, ou pour le génotypage des allèles de HLA-B*08 et HLA-DRB1*08, ou une altération quelconque dans le déséquilibre de liaison avec les gènes qui codifient lesdits antigènes.

13. Utilisation *in-vitro* de l'antigène HLA-B*08 en tant que marqueur pour l'analyse de la prédisposition génétique d'un individu à développer des effets défavorables suite à des implants biologiques non-métalliques implantés dans le corps.

14. Utilisation *in-vitro* des antigènes HLA-B*08 et HLA-DRB1*03 en tant que marqueurs pour l'analyse de la prédisposition génétique d'un individu à développer des effets défavorables suite à des implants biologiques non-métalliques implantés dans le corps.
